# EUROPÄISCHE PATENTSCHRIFT

(11) **EP 0 965 633 B1**
(45) Veröffentlichungstag und Bekanntmachung des Hinweises auf die Patenterteilung: **04.08.2004**
(21) Anmeldenummer: 99109208.1
(22) Anmeldetag: 20.05.1999
(51) Int. Cl.: C12M 3/00, C12M 3/04, C12M 1/24

(54) **Zellkulturgefäss für die Kultivierung nicht adhärenter Zellen**
Cell culture vessel for the cultivation of non-adherent cells
Récipient de culture de cellules pour la culture de cellules non adhérentes

(30) Priorität: 09.06.1998 DE 19825812
(43) Veröffentlichungstag der Anmeldung: 22.12.1999
(73) Patentinhaber: GSF - Forschungszentrum für Umwelt und Gesundheit GmbH, 85764 Oberschleissheim (DE)
(72) Erfinder: Reisbach, Gilbert, Dr., 80637 München (DE); Liu, Guoging, Dr., Chongching 630046 (CN)
(74) Vertreter: Reinhard - Skuhra - Weise & Partner

(56) Entgegenhaltungen:
- EP-A1- 0 014 007
- EP-A1- 0 552 412
- US-A- 4 693 983
- US-A- 5 151 366

## Beschreibung

Die Erfindung betrifft ein Zellkulturgefäß für die Kultivierung nicht adhärenter Zellen, insbesondere für die Lang- und Kurzzeitkultivierung primärer Zellen.

Für die Kultivierung derartiger Zellen ist es bisher üblich, diese in Mehrfachschalenplatten anzuordnen und mit Nährmedium zu versehen. Da dieses Nährmedium nach Verbrauch ausgetauscht werden muß, besteht das Problem, daß kultivierte nicht adhärente Zellen nicht in ihrer bisherigen Lage zurückgehalten werden, wodurch eine wechselseitige Nährwirkung benachbarter Zellen (autokriner, parakriner Effekt) eine deutliche Störung erfährt.

Mit der Erfindung soll ein neues Zellkulturgefäß für die Kultivierung nicht adhärenter Zellen verfügbar gemacht werden, das auch bei niedrigen Zelldichten beim Wechseln bzw. Abgießen des Nährmediums mit möglichst geringem Zellverlust und ohne Rückführung abgegossener Zellen eine Erhaltung der parakrinen bzw. autokrinen Nährfunktionen durch benachbarte Zellen ermöglicht.

Erfindungsgemäß wird diese Aufgabe durch die im Anspruch 1 genannten Merkmale gelöst.

Bevorzugte Merkmale, die die Erfindung vorteilhaft weiterbilden, sind den nachgeordneten Ansprüchen zu entnehmen.

Aufgrund der erfindungsgemäßen Ausgestaltung des neuen Zellkulturgefäßes fördert vorteilhaft der flache Bodenabschnitt mit den eingeformten asymmetrischen Vertiefungen das Überleben und Anwachsen primärer Kulturen durch Verstärkung autokriner und parakriner Mechanismen. Dieser Effekt ist beispielsweise für die Etablierung von humanen Tumorzellexplantaten von besonderer Bedeutung. Überdies halten die Vertiefungen des Bodenabschnitts abgesetzte nicht adhärente Zellen zurück und erlauben vorteilhaft ein Entnahme des Nährmediums ohne eine Verwirbelung und ohne benachbarte Zellen. Wirkungsvoll wird dadurch eine Rückführung der Zellen bei der Entnahme des verbrauchten Nährmediums in das Zellkulturgefäß und die damit verbundene Zentrifugation verhindert.

Gemäß einer bevorzugten Ausgestaltung der Erfindung fällt bei jeder Vertiefung die abgeschrägte Wand in Richtung auf die Seite der Ausgießöffnung zur gegenüberliegenden senkrechten Steilwand ab.

Die asymmetrischen Vertiefungen sind vorzugsweise regelmäßig angeordnet, insbesondere in Reihen, welche vorzugsweise jeweils gleichmäßig beabstandet nebeneinander vorgesehen sind.

Das Zellkulturgefäß ist als ein geschlossenes Gefäß ausgebildet und weist vorzugsweise die Form einer Flasche auf, wobei die Abgießöffnung im Flaschenhals und der Bodenabschnitt an einer flachen Flaschenwandseite gebildet ist.

Als weitere bevorzugte Maßnahme zum Vermeiden eines ungleichen Abfließens des verbrauchten Nährmediums ist weiterhin vorgesehen, daß zwischen den Vertiefungen und der Abgießöffnung ein Quersteg gebildet ist, welcher vorzugsweise unmittelbar vor dem Flaschenhals auf dem an einer flachen Seite der Flasche gebildeten Bodenabschnitt vorgesehen ist.

Hinsichtlich der geometrischen Ausgestaltung jeder Vertiefung ist nach einem bevorzugten Ausführungsbeispiel vorgesehen, daß die Größe der Vertiefung etwa 15 bis 20 µm und die innere Breite sowie die Länge der Vertiefung jeweils maximal etwa 500 µm betragen. Wenn zudem die schräge Wand den flachen Boden jeder Vertiefung etwa 20 µm vor der gegenüberliegenden Steilwand erreicht, wird vorteilhaft eine ebene Fläche von maximal 20 x 500 µm erreicht, und für einen ca. 3,7 cm breiten und 6,5 cm langen Zellkulturgefäßboden resultieren daraus ca. 60 Vertiefungen nebeneinander und ca. 130 Vertiefungen hintereinander, die vorzugsweise etwa um die Hälfte der Vertiefungsfläche - in Draufsicht gesehen - jeweils reihenweise zueinander versetzt sind. Benachbarte Reihen bilden somit vorzugsweise auch bei einer Vertiefungslänge von nur 100 µm eine seitliche Wand, die vorteilhaft eine Vermischung benachbarter Vertiefungsinhalte vermindert. Alternativ hierzu können bevorzugt die Vertiefungsreihen parallel mit einer schmalen Trennwand (ca. 5 µm Stärke, 15 bis 20 µm) zwischen den Reihen angeordnet sein, um ebenfalls eine seitliche Vermischung der Vertiefungsinhalte zu vermindern.

Selbstverständlich können statt der angegebenen Abmessungen auch kleinere Abmessungen zur Erreichung höherer Vertiefungszahlen vorgesehen sein, wodurch sich die Anzahl der abgesetzten Zellen in jeder Vertiefung verkleinert.

Nachfolgend wird die Erfindung unter Bezugnahme auf die beigefügten Zeichnungen näher erläutert. Es zeigen:
- Fig. 1: eine schematisierte Draufsicht auf den flachen Bodenabschnitt eines Zellkulturgefäßes gemäß der Erfindung; und
- Fig. 2: ein Schnitt entlang der Schnittlinie II/II in Fig. 1.

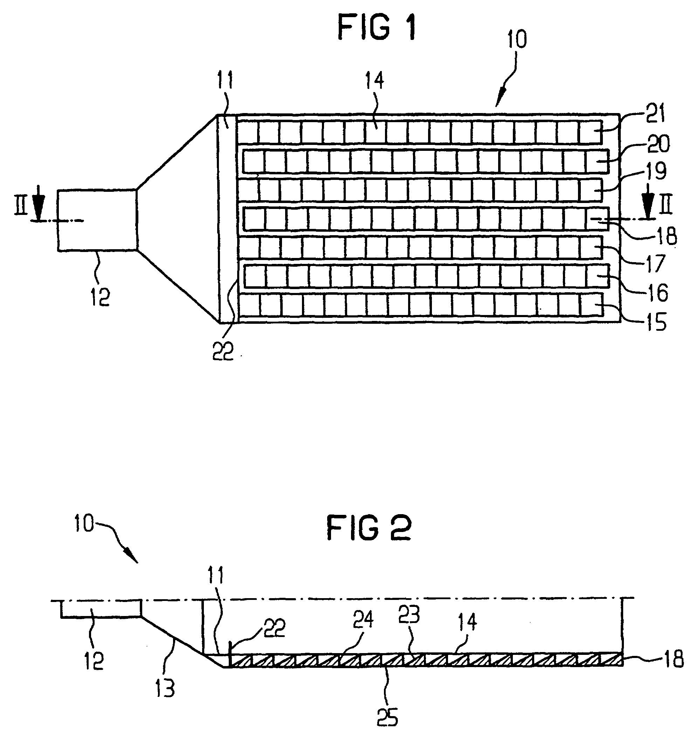

In Fig. 1 ist schematisiert und nicht maßstabsgetreu ein Zellkulturgefäß 10 in Ausbildung als Zellkulturflasche liegend in Draufsicht gezeigt. Das Zellkulturgefäß 10 besteht aus dem in Fig. 1 gezeigten flachen Bodenabschnitt 11, der bei der Zellkulturflasche gemäß Fig. 1 an einer flachen Seitenwand gebildet ist.

Über dem flachen Bodenabschnitt 11 erstreckt sich ein nicht dargestellter Oberabschnitt zur Herstellung bzw. Vervollständigung eines geschlossenen Zellkulturgefäßes, das nur eine seitliche Abgießöffnung 12 aufweist. Bei Ausbildung als Zellkulturflasche ist die seitliche Abgießöffnung 12 in dem Flaschenhals gebildet, wobei sich dieser Flaschenhals konisch über einen Abschnitt 13 zu dem Bodenabschnitt 11 erweitert.

In dem Bodenabschnitt 11 sind regelmäßig asymmetrische Vertiefungen 14 in zueinander versetzten Reihen 15 bis 21 angeordnet, die jeweils gleichmäßig beabstandet nebeneinander gebildet sind. Zwischen den Vertiefungen 14 und der Abgießöffnung 12 erstreckt sich von dem Bodenabschnitt 11 ein Quersteg 22, der zur Vermeidung eines ungleichen Abfließens von verbrauchten Nährmedium vorgesehen ist.

Jede Vertiefung 14 hat gemäß Fig. 1 in Draufsicht einen annähernd quadratischen Grundriß mit einer Seitenlänge von etwa 500 µm. Gemäß Fig. 2 ist die asymmetrische Gestaltung jeder Vertiefung 14 derart vorgesehen, daß jeweils eine in Richtung auf die Seite der Abgießöffnung 12 abfallende abgeschrägte Wand 23 vorgesehen ist, die etwa 20 µm vor einer gegenüberliegenden Steilwand 24 einen flachen Boden 25 erreicht. Der Versatz der asymmetrischen Vertiefungen benachbarter Reihen beträgt etwa 1/4 bis 1/2 der Länge der Vertiefung. Die Höhe des Steges 22 beträgt bis zu 2 mm und die Form des Stegs 22 ist gerade, in Richtung des Inneren der Zellkulturflasche geneigt oder leicht konvex, um ein gleichmäßiges Abfließen des Nährmediums zu ermöglichen. Die Winkel zwischen der Steilwand 24 und der angegrenzenden abgeschrägten Wand 23 sind abgerundet.

Das Zellkulturgefäß ist vorzugsweise aus Glas oder Kunststoff hergestellt und weist an der Unterseite des Bodenabschnitts vorzugsweise vier weiche, vibrationsdämpfende und wenige Millimeter große Gummiauflagen auf.

## Patentansprüche

1. Zellkulturgefäß für die Kultivierung nicht adhärenter Zellen, bestehend aus
einem flachen Bodenabschnitt (11),
einem sich darüber erstreckenden Oberabschnitt und aus einer seitlichen Abgießöffnung (12),
wobei in dem flachen Bodenabschnitt (11) asymmetrische Vertiefungen (14) mit einer annähernd senkrechten Steilwand (24) und einer gegenüberliegenden abgeschrägten Wand (23) gebildet sind.

2. Gefäß nach Anspruch 1, **dadurch gekennzeichnet, daß** jede abgeschrägte Wand (23) in Richtung auf die Seite der Abgießöffnung (12) abfällt.

3. Gefäß nach Anspruch 1 oder 2, **dadurch gekennzeichnet, daß** die asymmetrischen Vertiefungen (14) regelmäßig angeordnet sind.

4. Gefäß nach einem der vorangehenden Ansprüche, **dadurch gekennzeichnet, daß** die asymmetrischen Vertiefungen (14) in zueinander versetzten Reihen (15-21) angeordnet sind.

5. Gefäß nach Anspruch 4, **dadurch gekennzeichnet, daß** die Reihen (15-21) jeweils gleichmäßig beabstandet nebeneinander angeordnet sind.

6. Gefäß nach einem der vorangehenden Ansprüche, **dadurch gekennzeichnet, daß** das Zellkulturgefäß als Zellkulturflasche (10) ausgebildet ist.

7. Gefäß nach Anspruch 6, **dadurch gekennzeichnet, daß** die Abgießöffnung (12) im Flaschenhals gebildet ist.

8. Gefäß nach einem der vorangehenden Ansprüche, **dadurch gekennzeichnet, daß** zwischen den Vertiefungen (14) und der Abgießöffnung (12) ein Quersteg (22) zur Vermeidung eines ungleichen Abfließens von verbrauchten Nährmedium vorgesehen ist.

9. Gefäß nach Anspruch 8, **dadurch gekennzeichnet, daß** der Quersteg (22) unmittelbar vor dem Flaschenhals (12) gebildet ist.

10. Gefäß nach einem der vorangehenden Ansprüche, **dadurch gekennzeichnet, daß** die Größe jeder Vertiefung etwa 15 - 20 µm beträgt.

11. Gefäß nach einem der vorangehenden Ansprüche, **dadurch gekennzeichnet, daß** die innere Breite jeder Vertiefung maximal etwa 500 µm beträgt.

12. Gefäß nach einem der vorangehenden Ansprüche, **dadurch gekennzeichnet, daß** die Länge jeder Vertiefung (14) maximal etwa 500 µm beträgt.

13. Gefäß nach einem der vorangehenden Ansprüche, **dadurch gekennzeichnet, daß** die schräge Wand (23) den flachen Boden (25) jeder Vertiefung (14) etwa 20 µm vor der gegenüberliegenden Steilwand (24) erreicht.

## Claims

1. A cell culture vessel for the cultivation of non-adherent cells comprising:
- a flat bottom section (11);
- a top section extending beyond thereof; and
- a lateral spout orifice (12),
wherein asymmetric cavities (14) are formed in the flat bottom section (11) each cavity having a nearly perpendicular steep wall (24) and an opposing sloped wall (23).

2. The cell culture vessel according to claim 1, **characterized in that** each sloped wall (23) has a slope directing downwards to the side of the spout orifice (12).

3. The cell culture vessel according to claim 1 or 2, **characterized in that** the asymmetric cavities (14) are arranged regularly.

4. The cell culture vessel according to any one of the preceding claims, **characterized in that** the asymmetric cavities (14) are arranged in rows (15-21) staggered to each other.

5. A vessel according to claim 4, **characterized in that** the rows (15-21) are each arranged side by side at the same distance.

6. The vessel according to any one of the preceding claims, **characterized in that** the cell culture vessel is formed as a cell culture flask (10).

7. The vessel according to claim 6, **characterized in that** the spout orifice (12) is formed within a bottleneck.

8. The vessel according to any one of the preceding claims, **characterized in that** a cross web (22) is provided between the cavities (14) and the spout orifice (12) to prevent discontinuous draining of used nutrient.

9. The vessel according to claim 8, **characterized in that** the cross web (22) is formed directly in front of the bottleneck (12).

10. The vessel according to any one of the preceding claims, **characterized in that** the size of each cavity is about 15 to 20 µm.

11. The vessel according to any one of the preceding claims, **characterized in that** the internal width of each cavity is about 500 µm.

12. The vessel according to any one of the preceding claims, **characterized in that** the length of each cavity (14) is about 500 µm.

13. The vessel according to any one of the preceding claims, **characterized in that** the sloped wall (23) meets the flat bottom (25) of each cavity (14) at a distance of approximately 20 µm from the opposing steep wall (24).

## Revendications

1. Récipient de culture de cellules pour la culture de cellules non adhérentes, constitué
d'un tronçon de fond plat (11),
d'un tronçon supérieur s'étendant au-dessus, et d'un orifice de déversement latéral (12),
des renfoncements asymétriques (14), avec une paroi raide (24) approximativement verticale et une paroi inclinée (23) opposée, étant pratiqués dans le tronçon de fond plat (11).

2. Récipient selon la revendication 1, **caractérisé en ce que** chaque paroi inclinée (23) est inclinée vers le côté de l'orifice de déversement (12).

3. Récipient selon la revendication 1 ou 2, **caractérisé en ce que** les renfoncements asymétriques (14) sont disposés régulièrement.

4. Récipient selon l'une quelconque des revendications précédentes, **caractérisé en ce que** les renfoncements asymétriques (14) sont disposés en rangées (15 à 21) décalées les unes par rapport aux autres.

5. Récipient selon la revendication 4, **caractérisé en ce que** les rangées (15 à 21) sont respectivement disposées aux mêmes écartements les unes à côté des autres.

6. Récipient selon l'une quelconque des revendications précédentes, **caractérisé en ce que** le récipient de culture de cellules est agencé sous la forme d'une bouteille (10) de culture de cellules.

7. Récipient: selon la revendication 6, **caractérisé en ce que** l'orifice de déversement (12) est pratiqué dans le goulot de la bouteille.

8. Récipient selon l'une quelconque des revendications précédentes, **caractérisé en ce qu'**une entretoise transversale (22) est prévue entre les renfoncements (14) et l'orifice de déversement (12) afin d'éviter un écoulement irrégulier de milieu nourricier consommé.

9. Récipient selon la revendication 8, **caractérisé en ce que** l'entretoise transversale (22) est prévue directement devant le goulot (12) de la bouteille.

10. Récipient selon l'une quelconque des revendications précédentes, **caractérisé en ce que** la taille de chaque renfoncement est de l'ordre de 15 à 20 µm.

11. Récipient selon l'une quelconque des revendications précédentes, **caractérisé en ce que** la largeur intérieure de chaque renfoncement est de l'ordre de 500 µm maximum.

12. Récipient selon l'une quelconque des revendications précédentes, **caractérisé en ce que** la longueur de chaque renfoncement (14) est de l'ordre de 500 µm maximum.

13. Récipient selon l'une quelconque des revendications précédentes, **caractérisé en ce que** la paroi inclinée (23) atteint le fond plat (25) de chaque renfoncement (14) environ 20 µm avant la paroi raide (24) opposée.
